# EUROPEAN PATENT SPECIFICATION

(11) **EP 4 527 434 B1**
(45) Date of publication and mention of the grant of the patent: **28.10.2026**
(21) Application number: 23199146.4
(22) Date of filing: 22.09.2023
(51) Int. Cl.: A61M 5/32, A61M 5/50, A61M 5/31

(54) **SYRINGE NEEDLE SHIELD WITH TAMPER EVIDENCE FEATURE AND CONSISTENT NEEDLE PULL-OUT FORCE**
SPRITZENNADELSCHUTZ MIT MANIPULATIONSNACHWEISFUNKTION UND KONSISTENTER NADELAUSZUGSKRAFT
PROTECTION D'AIGUILLE DE SERINGUE AVEC CARACTÉRISTIQUE D'INVIOLABILITÉ ET FORCE D'EXTRACTION D'AIGUILLE CONSTANTE

(43) Date of publication of application: 26.03.2025
(73) Proprietor: Becton, Dickinson and Company, Franklin Lakes, NJ 07417-1880 (US)
(72) Inventor: EUVRARD, Nicolas, London SW17 0JF (GB)
(74) Representative: Germain Maureau

(56) References cited:
- EP-A2- 0 876 824
- US-A1- 2005 038 391
- US-A1- 2009 287 158
- US-A1- 2015 352 291

## Description

### BACKGROUND OF THE INVENTION

### Field of the Invention

The present disclosure relates generally to syringes and, more particularly, to a needle shield for a syringe, with the needle shield configured to provide tamper evidence and a consistent needle shield pull-out force.

### Description of Related Art

Syringes are used in a variety of environments for administering fluids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle and to preserve the sterility of the syringe content along with the syringe parts that come into contact with the patient skin.

Needle shields are generally formed to include a rigid component and a soft component. The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with an easily grippable surface for the user to remove the needle shield from the syringe.

With syringes, it is desirable for the needle shield to be removable in a predictable and consistent manner. That is, with existing syringes, it is recognized that the construction of the needle shield can contribute to the pull-out force required to remove the needle shield. As examples, the formulation of the soft component of the needle shield and/or the amount and location of contact between the soft component and the syringe barrel can contribute to the amount of pull-out force required to remove the needle shield.

With syringes, and in particular prefilled syringes, it is also important that integrity of the syringe is ensured, i.e. the integrity of the closure and thus of the fluid filled into the syringe. Ensuring the integrity of the syringe may be achieved by configuring the needle shield as a tamper-evident closure, with the needle shield including a means by which it may be determined whether the syringe has been tampered with, such as by the needle shield having been previously removed. In some embodiments, the needle shield may include a frangible web that is configured to break upon an initial removal of the needle shield, to indicate potential tampering with the syringe. However, it is recognized that problems with ensuring integrity of the syringe may occur if there is a mismatch between the pull-out force required to remove the needle shield and the force at which such a frangible web breaks to indicate tampering, i.e., if the required pull-out force is less than the force at which the frangible web breaks, as such a mismatch could potentially lead to the needle shield being removable without the frangible web breaking. It is known from the document US 2015/352291 a needle protection device. The document US 2009/287158 discloses an attachment for a syringe or cartridge. It is also known from the document US 2005/038391 a needle shield for a glass syringe.

Accordingly, a need exists in the art for a needle shield that exhibits a predictable and consistent pull-out force for removal thereof from the syringe. It is further desired that this pull-out force be aligned and matched with a tamper-evidence feature on the needle shield, such as the break-apart force required to break a frangible web, thereby ensuring that removal of the needle shield also results in breaking of the frangible web.

### SUMMARY OF THE INVENTION

Provided herein is a prefilled or prefillable syringe, the syringe comprising: a syringe assembly including: a syringe barrel having a barrel distal end and a barrel proximal end and defining a chamber, the syringe barrel including a tip member at the barrel distal end, the tip member comprising a hub portion having a distal end and a proximal end; a needle having a needle tip at a distal end of the needle and having a proximal end fixedly inserted into the tip member; and a needle shield having a shield distal end and a shield proximal end, with the shield proximal end secured to the tip member of the syringe barrel, so as to be positioned over the needle; and a plunger assembly received within the chamber of the syringe barrel and axially movable therein; wherein the needle shield comprises a rigid outer casing and a compliant inner casing positioned within the rigid outer casing, wherein the rigid outer casing includes a tamper evident frangible portion at the shield proximal end, the frangible portion secured to a main body portion of the rigid outer casing by a circumferential frangible web, and characterized in that the hub portion comprises a radial bump formed thereon at a location axially between the distal end and the proximal end and adjacent the proximal end of the hub portion, the radial bump having a hump-shaped construction and extending radially outward from a remainder of the hub portion, wherein the frangible portion includes a hook system that engages a proximally facing surface of the radial bump, to engage the needle shield with the syringe barrel, the hook system comprising a plurality of hooks formed at a proximal edge of the frangible portion and arranged circumferentially about an open proximal end of the outer casing, with the plurality of hooks angled distally and extending radially inward into an opening defined by the proximal edge of the frangible portion, with the hooks bending and sliding past the radial bump when the needle shield is pushed proximally over the needle and onto the tip member and with the hooks engaging the proximally facing surface of the radial bump to secure the needle shield in place.

In accordance with some aspects of the disclosure, the tip member of the syringe barrel comprises a hub portion having a diameter smaller than a cylindrical main portion of the syringe barrel extending out distally therefrom, the hub portion including the radial bump formed thereon and defining a channel in fluid communication with the chamber, with the needle secured within the channel.

In accordance with some aspects of the disclosure, the rigid outer casing comprises a tubular member including an open proximal end and an open distal end, with the main body portion and the frangible portion aligned axially to collectively form the tubular member, and with the frangible portion positioned proximally from the main body portion and affixed thereto by the circumferential frangible web.

In accordance with some aspects of the disclosure, the main body portion includes one or more windows formed therein adjacent the open distal end.

In accordance with some aspects of the disclosure, the compliant inner casing comprises an elongated body having a closed distal end and an open proximal end, the elongated body having an outer diameter smaller than an inner diameter of the tubular member to enable insertion of the compliant inner casing into the rigid outer casing through the open distal end of the tubular member, along with a collar member formed on the elongated body and extending radially outward therefrom, the collar positioned adjacent the closed distal end of the elongated body, wherein the collar member is at least partially retained within the one or more windows to secure the compliant inner casing within the rigid outer casing.

In accordance with some aspects of the disclosure, the elongated body includes a cavity formed therein, extending distally into the elongated body from the open proximal end thereof.

In accordance with some aspects of the disclosure, with the needle shield engaged with the syringe barrel, the needle extends distally through the cavity and into an elastomeric material of the compliant inner casing that is distal to the cavity.

In accordance with some aspects of the disclosure, the elongated body includes a tightness ring positioned at the open proximal end thereof, the tightness ring surrounding an opening of the cavity, and wherein the tightness ring engages a distal end of the hub portion to form a seal with the hub portion and prevent further proximal movement of the needle shield.

In accordance with some aspects of the disclosure, each of the tightness ring and the distal end of the hub portion includes a mating surface, the mating surfaces of the tightness ring and the hub portion engaging each other, with the mating surfaces comprising angled mating surfaces or flat mating surfaces.

In accordance with some aspects of the disclosure, engagement of the tightness ring with the distal end of the hub portion, along with engagement of the needle with the compliant inner casing, generates a distally directed force that pushes the collar member up against a distal edge of the one or more windows.

In accordance with some aspects of the disclosure, the tightness ring is positioned within the main body portion of the rigid outer casing such that the frangible portion of the rigid outer casing is proximal from the compliant inner casing.

In accordance with some aspects of the disclosure, the tightness ring of the elongated body is separated axially from the radial bump of tip member and from a radial part of the hub portion.

In accordance with some aspects of the disclosure, the elongated body is tapered from the closed distal end to the open proximal end thereof.

In accordance with some aspects of the disclosure, a pull-out force required to remove the needle shield from the syringe barrel and off the needle is equal to a force required to break the circumferential frangible web, such that the circumferential frangible web breaks upon removal of the needle shield from the syringe barrel, leaving the frangible portion on the syringe barrel.

### BRIEF DESCRIPTION OF THE DRAWINGS

FIG. 1 is a perspective view of a syringe including a needle shield, according to an embodiment of the disclosure;
FIG. 2 is an exploded view of the syringe of FIG. 1;
FIG. 3 is an enlarged front view of an outer casing of the needle shield included in the syringe of FIG. 1, according to an embodiment of the disclosure;
FIG. 4 is a cross-sectional view of the outer casing of FIG. 3;
FIG. 5 is an enlarged front view of an inner casing of the needle shield included in the syringe of FIG. 1, according to an embodiment of the disclosure;
FIG. 6 is a cross-sectional view of the inner casing of FIG. 5;
FIG. 7 is a cross-sectional view taken along line 7-7 of FIG. 1;
FIGS. 8A, 8B and 8C illustrate process steps for assembling the needle shield included in the syringe of FIG. 1, according to an embodiment of the disclosure; and
FIGS. 9A and 9B illustrate process steps for detaching the needle shield of FIG. 1, according to an embodiment of the disclosure.

### DESCRIPTION OF THE INVENTION

The following description is provided to enable those skilled in the art to make and use the described embodiments contemplated for carrying out the invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

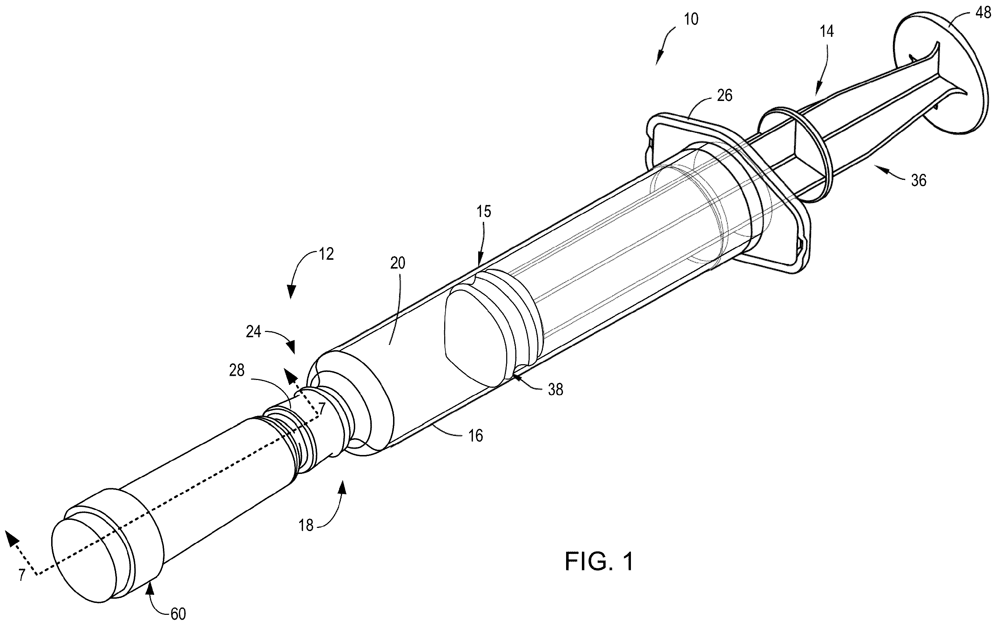

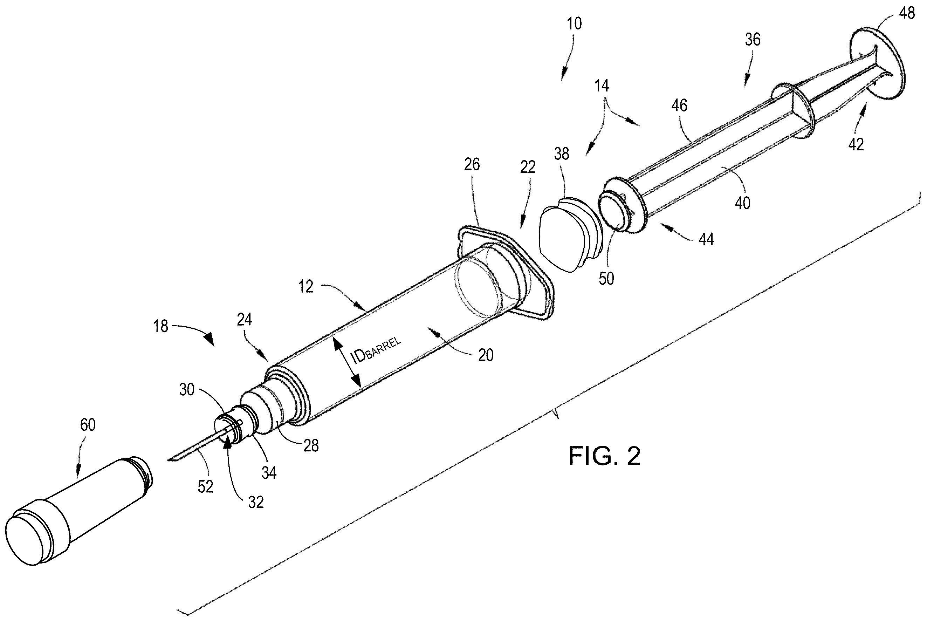

Referring to FIGS. 1 and 2, shown is a non-limiting embodiment of a medical injection device 10, such as a prefilled syringe for example, with which aspects or embodiments of the disclosure may be implemented. While the medical injection device is shown and described hereafter as a syringe 10, it is recognized that other medical injection devices may incorporate aspects of the disclosure, as described in detail here below.

As shown in FIGS. 1 and 2, the syringe 10 generally includes a syringe assembly 12 (i.e., a barrel with a puncture needle mounted with a shield) and a plunger assembly 14. The plunger assembly 14 is movable within the syringe assembly 12 along a longitudinal axis to an advanced position to facilitate administering of an injectable fluid (e.g., medication) to a patient, for example.

The syringe assembly 12 includes a syringe barrel 15 formed of a generally cylindrical outer wall 16 and a tip member 18 that collectively define a chamber 20 for retaining fluid therein. The syringe barrel 15 includes an open proximal end 22 configured to receive the plunger assembly 14 therein and a distal end 24 at which tip member 18 is positioned. The proximal end 22 of the syringe barrel 15 may include a flange 26 to facilitate handling and positioning of the syringe 10 and to maintain the relative position of the syringe barrel 15 with respect to the plunger assembly 14 during medication administration. At the distal end 24, the tip member 18 may include a hub portion 30 that is formed as a partially hollow member that defines a channel 32 therethrough in fluid communication with the chamber 20. The hub portion 30 extends distally from the syringe barrel 15 and has a reduced diameter as compared to the main body portion of the syringe barrel 15 (i.e., the cylindrical portion formed by outer wall 16). In some embodiments, the tip member 18 may also include a shoulder 28 positioned between the cylindrical outer wall 16 and the hub portion 30, with the shoulder 28 having an intermediate diameter that provides a transition between the cylindrical outer wall 16 and the hub portion 30 - but in other embodiments the shoulder 28 may be omitted.

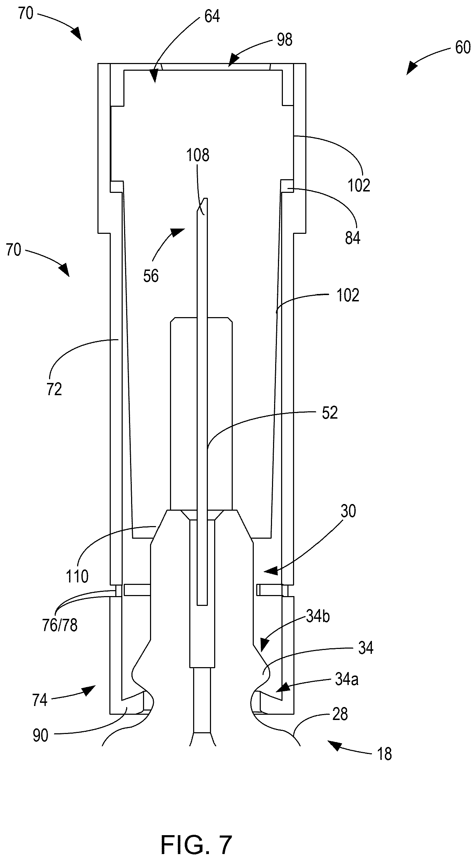

As shown in FIG. 2, according to aspects of the disclosure, the hub portion 30 includes a bump 34 formed thereon that extends radially outward from a remainder of the hub portion 30. The bump 34 is formed at an axial location of the hub portion 30 that is between distal and proximal ends thereof, with the bump 34 positioned near or adjacent the proximal end of the hub portion 30 - such that the bump 34 is positioned near the shoulder 28 (or near the cylindrical outer wall 16, if no shoulder 28). In some embodiments, the radial bump 34 may have a generally rounded or hump-shaped construction, but may include a proximal facing surface 34a of the bump 34 (see FIG. 7) that presents a generally flat (or less sloped) surface, so as to allow for engagement of a needle shield therewith, as explained in greater detail below.

The plunger assembly 14 of syringe 10 is formed of an elongate plunger rod 36 and a plunger head or stopper 38. The plunger rob 36 may include a main body 40 extending between a plunger rod proximal end 42 and a plunger rod distal end 44. In some embodiments, the main body 40 may include a plurality of elongate vanes or walls 46 extending axially along a length thereof between the plunger rod proximal end 42 and the plunger rod distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 15. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger rod 36 within the chamber 20 of syringe barrel 15. The stopper 38 may be made from a material that is different from the material of the plunger rod 36 and that is capable of forming a tight seal with the syringe barrel 15 as it is advanced therethrough. In some embodiments, the stopper 38 includes a receptacle (not shown) formed therein that is sized and configured to receive the flanged extension member 50 of plunger rod 36, with the flanged extension member 50 coupling with the receptacle via a press fit connection, for example, to secure the stopper 38 to the plunger rod 36, although it can be appreciated that the stopper 38 and plunger distal end 44 can be secured by other techniques known in the art.

As indicated above, in addition to syringe barrel 15, syringe assembly 12 also includes a needle 52 that is secured to the syringe barrel 15. The needle 52 of syringe assembly 12 is attached to the hub portion 30 within channel 32, with a proximal end 54 of the needle 52 positioned within the channel 32 and a distal end 56 of the needle 52 extending out from the hub portion 30 (see FIG. 7). The proximal end 54 of the needle 52 may be inserted into the channel 32 of hub portion 30 and may be fixed therein with an adhesive (e.g., glue), by thermal welding, or the like. The needle 52 defines a hollow lumen therein that is in fluid communication with the chamber 20 of syringe barrel 15, such that fluid may be dispensed from the syringe 10.

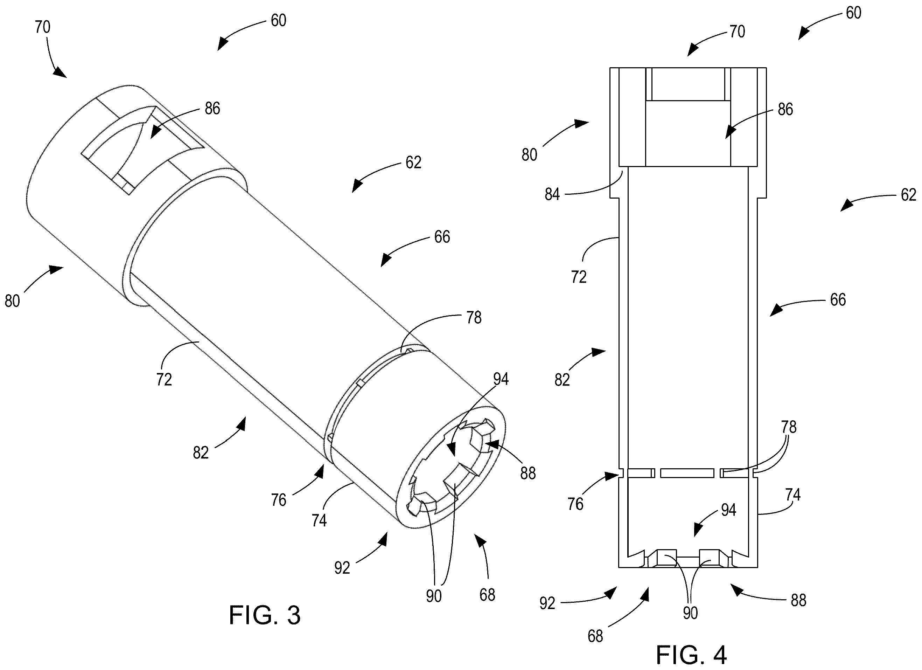

As shown in FIGS. 1 and 2, and now also in FIGS. 3-7, a needle shield 60 of syringe assembly 12 may be coupled to the syringe barrel 15 to protect the needle 52. According to aspects of the disclosure, the needle shield 60 may be composed of a rigid outer casing 62 that provides structure to the needle shield 60 and a compliant inner casing 64 that surrounds the needle 52 (when needle shield 60 is coupled to syringe barrel 15). In various embodiments, the outer casing 62 may be made of a rigid material such as polypropylene (PP), polyethylene (PE), polyethylene terephthalate (PET), polystyrene (PS) or polycarbonate (PC), while the inner casing 64 is made of a deformable material such as rubber (e.g., butyl rubber, isoprene rubber, latex rubber, silicone rubber) or a thermoplastic elastomer (TPE), as non-limiting examples. The outer casing 62 and inner casing 64 of needle shield 60 are formed as separate components that may be secured together to form the needle shield 60, with the casings 62, 64 formed via separate manufacturing processes - such as the outer casing 62 being formed via injection molding and the inner casing 64 being formed via injection molding (if TPE) or compression molding (if rubber).

As shown in FIGS. 3 and 4 and FIG. 7, the outer casing 62 is constructed as a tubular member 66 that includes an open proximal end 68 and an open distal end 70, with the outer casing 62 including a main body portion 72 and a frangible portion 74 that are axially aligned to form the tubular member 66. The main body portion 72 composes the open distal end 70 of the tubular member 66, while the frangible portion 74 composes the open proximal end 68 of the tubular member 66. The frangible portion 74 is configured to break off from the main body portion 72 responsive to an axial pulling force being applied to the main body portion 72, with the frangible portion 74 secured to the main body portion 72 of the rigid outer casing 62 by a circumferential frangible web 76. The frangible web 76 may be composed of a plurality of circumferentially spaced-apart web pieces 78 of narrow diameter/thickness each configured to break when an axial pulling force of sufficient strength is applied thereto. Accordingly, the frangible portion 74 of outer casing 62 - when broken off from main body portion 72 - functions as a tamper evidence feature that indicates if the needle shield 60 has been previously removed from syringe 10.

The main body portion 72 is generally tubular in shape, but includes a distal first portion 80 of greater diameter than a proximal second portion 82 thereof. The distal first portion 80 is stepped out from the proximal second portion 82, such that a ledge or step 84 is formed on an inner surface of the main body portion 72 between the distal first portion 80 and the proximal second portion 82. The distal first portion 80 is further configured to include one or more windows 86 formed therein, adjacent the open distal end 70 of the outer casing 62. The window(s) 86 are useful to facilitate the permeation of gas during gas sterilization (e.g. EtO, steam) of the syringe 10 during manufacturing thereof, and are further configured to receive/retain portions of the inner casing 64 therein in order to retain the inner casing 64 within the outer casing 62, as explained in further detail below.

With reference again now to the frangible portion 74 of outer casing 62, the frangible portion 74 includes a hook system 88 thereon that functions to axially secure the needle shield 60 to the syringe barrel 15. The hook system 88 is formed of a plurality of hooks 90 that are positioned at a proximal edge 92 of the frangible portion 74 and arranged circumferentially about the open proximal end 68 of outer casing 62. The plurality of hooks 90 extend radially inward into an opening 94 defined by the proximal edge 92 of the frangible portion 74. The plurality of hooks 90 are angled distally and are configured to flex and bend when a distally directed pressure is applied thereto, but are configured so as to not flex and bend when a proximally directed pressure is applied thereto. The hooks 90 are arranged and positioned such that they may interact with the radial bump 34 on hub portion 30. That is, the hooks 90 allow for the needle shield 60 to be secured onto the syringe barrel 15, with the hooks 90 bending and sliding past bump 34 when the needle shield 60 is pushed proximally over needle 52 and onto tip member 18 of syringe barrel 15, and with the hooks 90 engaging the proximally facing surface 34a of the radial bump 34 to secure the needle shield 60 in place.

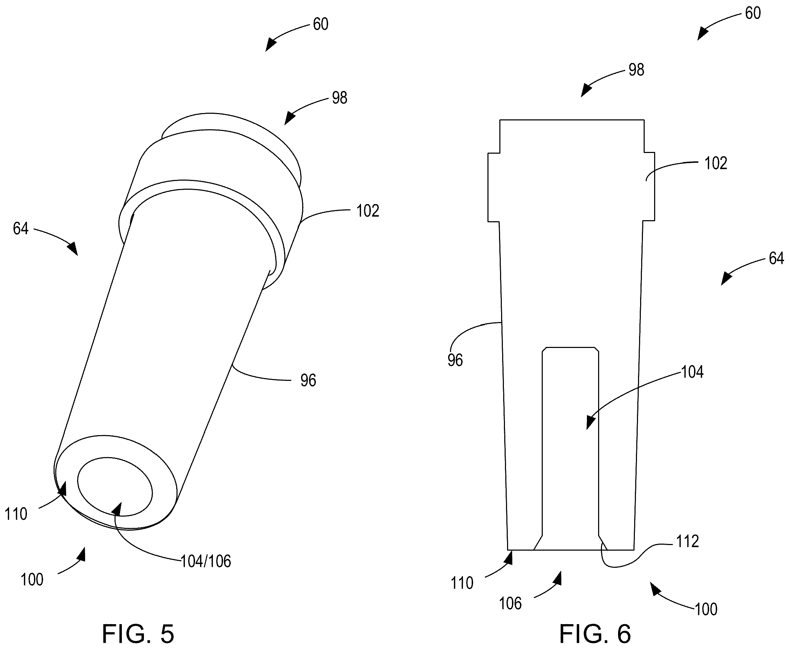

As shown in FIGS. 5, 6, and 7, the inner casing 64 includes an elongated body 96 having a closed distal end 98 and an open proximal end 100. In some embodiments, the elongated body 96 may be formed as a tapered body having a diameter that reduces distally-to-proximally - i.e., is tapered from the closed distal end 98 to the open proximal end 100. The elongated body 96 (at the larger distal end) has an outer diameter that is smaller than an inner diameter of the open distal end 70 of outer casing 62, so as to enable insertion of the inner casing 64 into the rigid outer casing 62 through the open distal end 70 thereof. A collar member 102 is formed on the elongated body 96 adjacent the closed distal end 98 thereof. The collar member 102 extends radially outward therefrom, so as to present a feature on the inner casing 64 of increased diameter. When the inner casing 64 is positioned within the outer casing 62, the collar member 102 is configured so as to be at least partially retained within the window(s) 86 of the main body portion 72, to aid in securing the compliant inner casing 64 within the rigid outer casing 62.

A hollow cavity 104 is formed in the elongated body 96 at the open proximal end 100 thereof, with an opening 106 in the elongated body 96 providing access into the cavity 104. The cavity 104 extends distally into the elongated body 96 a portion of the length of the elongated body 96. The cavity 104 may have an inner diameter that is smaller than an outer diameter of the hub portion 30, such that the hub portion 30 is prevented from sliding into cavity 104 when the needle shield 60 is secured to syringe barrel 15. The cavity 104 is sized and positioned to receive at least a portion of the needle 52 therein, to provide protection to the needle 52 once the needle shield 60 is coupled to the syringe barrel 15. In some embodiments, the cavity 104 is configured such that, when the needle shield 60 is secured on syringe barrel 15, the needle 52 extends through a length of the cavity 104, such that a needle tip 108 (see FIG. 7) protrudes through the cavity 104 to reach the compliant material of closed distal end 98 that is positioned distally from cavity 104 - i.e., the needle tip 108 punctures and extends into the compliant material - such that the needle tip 108 is sealed in the compliant material of the inner casing 64. With the needle tip 108 sealed in the compliant material of the inner casing 64, leakage of fluid out from the needle 52 may be prevented.

Provided at the open proximal end 100 of the elongated body 96 is an annular member that may be termed a tightness ring 110. The tightness ring 110 surrounds the opening 106 of cavity 104 and provides an engagement surface at which the inner casing 64 may be brought into contact with the tip member 18 of syringe barrel 15 - i.e., with hub portion 30. In accordance with some aspects of the disclosure, the tightness ring 110 may include an angled mating surface 112, with the angled mating surface 112 of the tightness ring 110 engaging an angled mating surface at the distal end of the hub portion 30. In other embodiments, the tightness ring 110 may include a flat mating surface 112, with the flat mating surface 112 of the tightness ring 110 engaging a flat mating surface at the distal end of the hub portion 30. The mating/sealing of the tightness ring 110 with distal end of the hub portion 30 provides a seal that prevents contamination ingress into the cavity 104 and may also prevent a leakage of fluid (from the needle 52) out from the needle shield 60. The mating/sealing of the tightness ring 110 with distal end of the hub portion 30 also functions as a stop that prevents further proximal movement of the needle shield 60 (i.e., prevents sliding of hub portion 30 further into cavity 104).

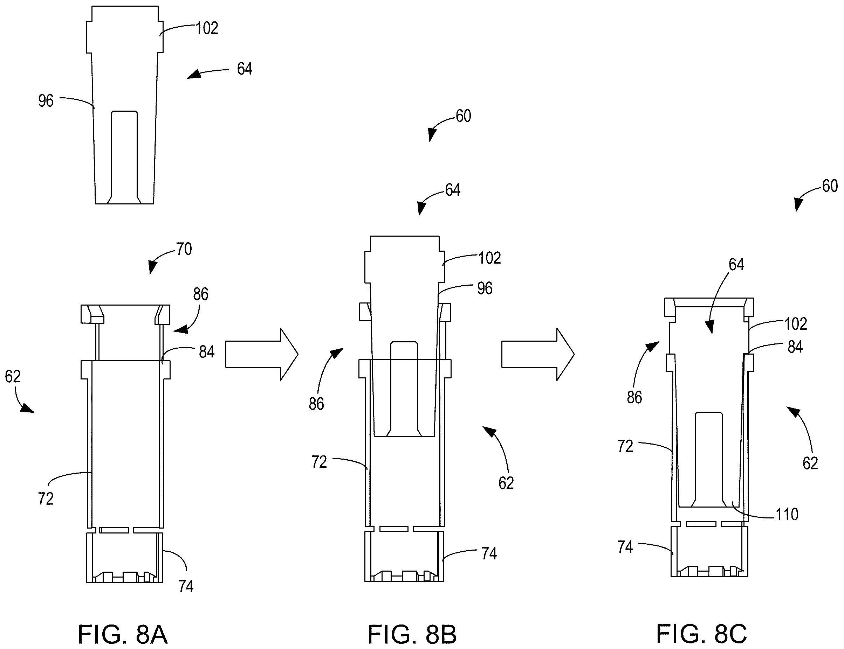

Referring now to FIGS. 8A, 8B, and 8C, and with continued reference to FIGS. 1-7, assembly of the needle shield 60 and mating thereof with tip member 18 of syringe barrel 15 is described in further detail. In initially assembling the needle shield 60, the compliant inner casing 64 is introduced into the rigid outer casing 62. The elongated body 96 of inner casing 64 is positioned within the open distal end 70 of outer casing 62 and advanced proximally therethrough. The elongated body 96 of inner casing 64 is advanced distally through the main body portion 72 of outer casing 62 until the collar member 102 is brought into axially alignment with the window(s) 86 of the main body portion 72. With the collar member 102 axially aligned with the window(s) 86, the collar member 102 engages the window(s) 86 and also abuts the ledge or step 84 formed on main body portion 72 - such that further proximal movement of the inner casing 64 into outer casing 62 is prevented and the inner casing 64 is secured in place within the outer casing 62. As shown in FIG. 8C, with the inner casing 64 secured in place within the outer casing 62, the tightness ring 110 of inner casing 64 is positioned within the main body portion 72 of the rigid outer casing 62, such that the frangible portion 74 of the rigid outer casing 62 is proximal from the compliant inner casing 64.

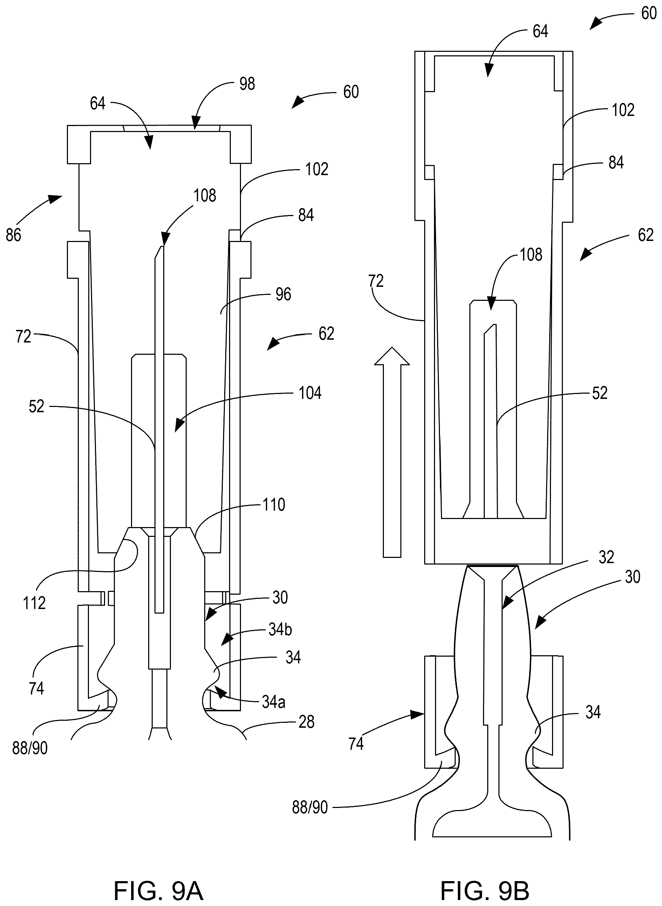

Referring now to FIG. 9A, with the needle shield 60 assembled as described above, the needle shield 60 may be secured to the tip member 18 of syringe barrel 15 and to needle 52. In engaging the needle shield 60 with the tip member 18 and needle 52, the needle shield 60 is positioned such that the cavity 104 in inner casing 64 is aligned with the needle 52. The needle shield 60 is then moved in the proximal direction, such that needle 52 enters into cavity 104 and moves therethrough until the needle tip 108 is pressed into the compliant material of elongated body 96 distal of the cavity 104, near the closed distal end 98 of inner casing 64.

As the needle shield 60 is moved down over needle 52, the hook system 88 is brought into contact with radial bump 34 on hub portion 30. As described previously, as the needle shield 60 is advanced proximally and the hook system 88 brought into contact with radial bump 34, a proximally directed pressure is applied to the hooks 90 that causes the hooks 90 to flex and bend, so as to allow the hooks 90 to slide over a distally facing surface 34b of the bump 34 and be pushed proximally past the bump 34. Upon moving proximally past the bump 34, the hooks 90 then engage the proximally facing surface 34a of the radial bump 34 to lock the hooks 90 on the bump 34 and secure the needle shield 60 in place on tip member 18. Once locked with the bump 34, the hooks 90 permanently secure the frangible portion 74 of outer casing 62 to hub portion 30, as the hooks 90 cannot be moved distally past the bump 34.

As the hooks 90 are moved proximally past the bump 34 and into engagement with the proximally facing surface 34a thereof, the tightness ring 110 is also brought into contact with the distal end of hub portion 30. As described above, each of the tightness ring 110 and the distal end of the hub portion 30 may include an mating surface that may be configured as angled surface or flat surface, with the mating surfaces of the tightness ring 110 and the hub portion 30 engaging each other to seat the hub portion 30 in the tightness ring 110. The mating/sealing of the tightness ring 110 with distal end of the hub portion 30 provides a seal that prevents contamination ingress into the cavity 104 and also functions as a stop that prevents further proximal movement of the needle shield 60 (i.e., prevents sliding of hub portion 30 further into cavity 104).

In accordance with some aspects of the disclosure, engagement of the tightness ring 110 with the distal end of the hub portion 30 as described above - in combination with engagement of the needle 52 into/with the compliant material that forms inner 64 (as the needle protrudes through cavity 104 and into the compliant material - generates a distally directed force that pushes the collar member 102 of inner casing 64 back up against a distal edge of the window(s) 86 in outer casing 62. This force ensures continued/secure engagement between the inner casing 64 and outer casing 62.

It is noted that, with the needle shield 60 secured on tip member 18 and needle 52 as described above, the compliant inner casing 64 (and, specifically, the tightness ring 110 provided at the proximal end of the elongated body 96 thereof) is separated axially from the radial bump 34 of tip member 18 and from a radial part of the hub portion 30. With this separation provided, any interaction and engagement between the compliant material of the inner casing 64 and the bump 34 and/or between the compliant material of the inner casing 64 and the radial part of the hub portion 30 is prevented, which is desirable as such interaction could affect the pull-out force required to remove the needle shield 60 from the tip member 18 of syringe barrel 15 when desired. Accordingly, the pull-out force can be kept low (i.e., lower than if inner casing 64 is engaged with bump 34) and to a consistent/predictable level that is not influenced by properties of the compliant material from which inner casing 64 is formed, and how those materials might engage/interact if engaged with the bump 34.

With engagement of the needle shield 60 to the tip member 18 and needle 52 as described above, removal of the needle shield 60 is also controlled in a desired manner. In removing the needle shield 60 from the tip member 18 and needle 52 of syringe 10, a distally directed axial pulling force may be applied to the needle shield 60, such as by a healthcare provider grasping the main body portion 72 of outer casing 62 and pulling distally on the outer casing 62. With the hooks 90 of the frangible portion 74 of outer casing 62 locked onto the bump 34 of tip member 18, it is recognized that the axial pull-out force required to remove the needle shield 60 is equal to the axial force necessary to break the frangible web 76 connecting the frangible portion 74 of outer casing 62 to the main body portion 72 of outer casing 62. That is, the needle shield 60 can only be removed upon breaking of the frangible web 76. With the needle shield 60 configured as such, the needle shield 60 is configured as a tamper evident shield - as any removal of the shield 60 results in separation of the frangible portion 74 from the main body portion 72, such that even if the needle shield 60 is later repositioned on the syringe 10, the frangible portion 74 will remain separated from the main body portion 72, as evidence of previous tampering with the syringe 10.

Beneficially, embodiments of the invention thus provide a needle shield that is configured to provide tamper evidence and a consistent needle pull-out force. The needle shield includes a rigid outer casing having a frangible portion that is removable from a remainder of the needle shield (i.e., from a main body portion of the outer casing and from a compliant inner casing). The needle shield is constructed such that the axial pull-out force required to remove the needle shield from the remainder of the syringe is equal to the axial force necessary to break the frangible portion off from the main body portion, such that the frangible portion will break off anytime the needle shield is removed from the syringe.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications that are within the scope of the appended claims. For example, it is to be understood that the present disclosure contemplates that, to the extent possible, one or more features of any embodiment may be combined with one or more features of any other embodiment.

## Claims

1. A prefilled or prefillable syringe (10), the syringe (10) comprising:
a syringe assembly (12) including:
a syringe barrel (15) having a barrel distal end (24) and a barrel proximal end (22) and defining a chamber (20), the syringe barrel (15) including a tip member (18) at the barrel distal end (24), the tip member (18) comprising a hub portion (30) having a distal end and a proximal end;
a needle (52) having a needle tip at a distal end of the needle (52) and having a proximal end fixedly inserted into the tip member (18); and
a needle shield (60) having a shield distal end and a shield proximal end, with the shield proximal end secured to the tip member (18) of the syringe barrel (15), so as to be positioned over the needle (52); and
a plunger assembly (14) received within the chamber (20) of the syringe barrel (15) and axially movable therein;
wherein the needle shield (60) comprises a rigid outer casing (62) and a compliant inner casing (64) positioned within the rigid outer casing (62),
wherein the rigid outer casing (62) includes a tamper evident frangible portion (74) at the shield proximal end, the frangible portion (74) secured to a main body portion (72) of the rigid outer casing (62) by a circumferential frangible web (76), and
**characterized in that** the hub portion (30) comprises a radial bump (34) formed thereon at a location axially between the distal end and the proximal end and adjacent the proximal end of the hub portion (30), the radial bump (34) having a hump-shaped construction and extending radially outward from a remainder of the hub portion (30), wherein the frangible portion (74) includes a hook system (88) that engages a proximally facing surface (34a) of the radial bump (34), to engage the needle shield (60) with the syringe barrel (15), the hook system (88) comprising a plurality of hooks (90) formed at a proximal edge of the frangible portion (74) and arranged circumferentially about an open proximal end (68) of the outer casing (62), with the plurality of hooks (90) angled distally and extending radially inward into an opening (94) defined by the proximal edge of the frangible portion (74), with the hooks (90) bending and sliding past the radial bump (34) when the needle shield (60) is pushed proximally over the needle (52) and onto the tip member (18) and with the hooks (90) engaging the proximally facing surface (34a) of the radial bump (34) to secure the needle shield (60) in place.

2. The syringe (10) of claim 1, wherein the rigid outer casing (62) comprises a tubular member (66) including an open proximal end and an open distal end, with the main body portion (72) and the frangible portion (74) aligned axially to collectively form the tubular member (66), and with the frangible portion (74) positioned proximally from the main body portion (72) and affixed thereto by the circumferential frangible web (76).

3. The syringe (10) of claim 2, wherein the main body portion (72) includes one or more windows (86) formed therein adjacent the open distal end.

4. The syringe (10) of claim 3, wherein the compliant inner casing (64) comprises:
an elongated body (96) having a closed distal end and an open proximal end, the elongated body (96) having an outer diameter smaller than an inner diameter of the tubular member (66) to enable insertion of the compliant inner casing (64) into the rigid outer casing (62) through the open distal end of the tubular member (66); and
a collar member (102) formed on the elongated body (96) and extending radially outward therefrom, the collar positioned adjacent the closed distal end of the elongated body (96);
wherein the collar member (102) is at least partially retained within the one or more windows (86) to secure the compliant inner casing (64) within the rigid outer casing (62).

5. The syringe (10) of claim 4, wherein the elongated body (96) includes a cavity (104) formed therein, extending distally into the elongated body (96) from the open proximal end thereof.

6. The syringe (10) of claim 5, wherein with the needle shield (60) engaged with the syringe barrel (15), the needle (52) extends distally through the cavity (104) and into an elastomeric material of the compliant inner casing (64) that is distal to the cavity (104).

7. The syringe (10) of claim 5, wherein the elongated body (96) includes a tightness ring (110) positioned at the open proximal end thereof, the tightness ring (110) surrounding an opening of the cavity (104).

8. The syringe (10) of claim 7, wherein the hub portion (30) has a diameter smaller than a cylindrical main portion of the syringe barrel (15) extending out distally therefrom, the hub portion (30) including the radial bump (34) formed thereon and defining a channel (32) in fluid communication with the chamber (20), with the needle (52) secured within the channel (32), and wherein the tightness ring (110) engages a distal end of the hub portion (30) to form a seal with the hub portion (30) and prevent further proximal movement of the needle shield (60).

9. The syringe (10) of claim 8, wherein each of the tightness ring (110) and the distal end of the hub portion (30) includes a mating surface, the mating surfaces of the tightness ring (110) and the hub portion (30) engaging each other, with the mating surfaces comprising angled mating surfaces or flat mating surfaces.

10. The syringe (10) of claim 8 or claim 9, wherein engagement of the tightness ring (110) with the distal end of the hub portion (30), along with engagement of the needle (52) with the compliant inner casing (64), generates a distally directed force that pushes the collar member (102) up against a distal edge of the one or more windows (86).

11. The syringe (10) of any of claims 7-10, wherein the tightness ring (110) is positioned within the main body portion (72) of the rigid outer casing (62) such that the frangible portion (74) of the rigid outer casing (62) is proximal from the compliant inner casing (64).

12. The syringe (10) of any of claims 7-11, wherein the tightness ring (110) of the elongated body (96) is separated axially from the radial bump (34).

13. The syringe (10) of any of claims 4-12, wherein the elongated body (96) is tapered from the closed distal end to the open proximal end thereof.

14. The syringe (10) of any of claims 1-13, wherein a pull-out force required to remove the needle shield (60) from the syringe barrel (15) and off the needle (52) is equal to a force required to break the circumferential frangible web (76), such that the circumferential frangible web (76) breaks upon removal of the needle shield (60) from the syringe barrel (15), leaving the frangible portion (74) on the syringe barrel (15).

## Patentansprüche

1. Vorgefüllte oder vorfüllbare Spritze (10), wobei die Spritze (10) umfasst:
eine Spritzenbaugruppe (12), die enthält:
einen Spritzenzylinder (15), der ein distales Ende (24) und ein proximales Ende (22) des Zylinders aufweist und eine Kammer (20) definiert, wobei der Spritzenzylinder (15) ein Spitzenelement (18) am distalen Ende (24) des Zylinders enthält, wobei das Spitzenelement (18) einen Nabenabschnitt (30) umfasst, der ein distales Ende und ein proximales Ende aufweist;
eine Nadel (52), die eine Nadelspitze an einem distalen Ende der Nadel (52) aufweist und ein proximales Ende aufweist, das fest in das Spitzenelement (18) eingeführt ist; und
einen Nadelschutz (60), der ein distales Schutzende und ein proximales Schutzende aufweist, wobei das proximale Schutzende am Spitzenelement (18) des Spritzenzylinders (15) befestigt ist, so dass es über der Nadel (52) positioniert ist; und
eine in der Kammer (20) des Spritzenzylinders (15) aufgenommene und darin axial bewegliche Kolbenbaugruppe (14);
wobei der Nadelschutz (60) ein starres äußeres Gehäuse (62) und ein konformes inneres Gehäuse (64) umfasst, das innerhalb des starren äußeren Gehäuses (62) positioniert ist,
wobei das starre äußere Gehäuse (62) einen manipulationssicheren Bruchabschnitt (74) am proximalen Schutzende enthält, wobei der Bruchabschnitt (74) durch einen umlaufenden Bruchsteg (76) an einem Hauptkörperabschnitt (72) des starren äußeren Gehäuses (62) befestigt ist, und
**dadurch gekennzeichnet, dass** der Nabenabschnitt (30) eine daran axial zwischen dem distalen Ende und dem proximalen Ende und angrenzend am proximalen Ende des Nabenabschnitts (30) gebildete radiale Wölbung (34) umfasst, wobei die radiale Wölbung (34) eine gewölbte Konstruktion aufweist und sich radial nach außen von einem Rest des Nabenabschnitts (30) erstreckt, wobei der Bruchabschnitts (74) ein Hakensystem (88) enthält, das in eine proximal zugewandte Oberfläche (34a) der radialen Wölbung (34) eingreift, damit der Nadelschutz (60) in den Spritzenkörper (15) eingreift, wobei das Hakensystem (88) eine Vielzahl von Haken (90) umfasst, die an einer proximalen Kante des Bruchabschnitts (74) gebildet und umlaufend um ein offenes proximales Ende (68) des äußeren Gehäuses (62) angeordnet sind, wobei die Vielzahl von Haken (90) distal abgewinkelt sind und sich radial nach innen in eine Öffnung (94) erstrecken, die durch die proximale Kante des Bruchabschnitts (74) definiert ist, wobei die Haken (90) sich biegen und über die radiale Wölbung (34) gleiten, wenn der Nadelschutz (60) proximal über die Nadel (52) und auf das Spitzenelement (18) geschoben wird, wobei die Haken (90) in die proximal zugewandte Oberfläche (34a) der radialen Wölbung (34) eingreifen, um den Nadelschutz (60) zu sichern.

2. Spritze (10) nach Anspruch 1, wobei das starre äußere Gehäuse (62) ein röhrenförmiges Element (66) umfasst, das ein offenes proximales Ende und ein offenes distales Ende umfasst, wobei der Hauptkörperabschnitt (72) und der Bruchabschnitt (74) axial ausgerichtet sind, um gemeinsam das röhrenförmige Element (66) zu bilden, und wobei der Bruchabschnitt (74) proximal vom Hauptkörperabschnitt (72) positioniert und daran durch den umlaufenden Bruchsteg (76) befestigt ist.

3. Spritze (10) nach Anspruch 2, wobei der Hauptkörperabschnitt (72) ein oder mehrere darin neben dem offenen distalen Ende gebildete Fenster (86) enthält.

4. Spritze (10) nach Anspruch 3, wobei das konforme innere Gehäuse (64) umfasst:
einen länglichen Körper (96), der ein geschlossenes distales Ende und ein offenes proximales Ende aufweist, wobei der längliche Körper (96) einen Außendurchmesser aufweist, der kleiner ist als ein Innendurchmesser des röhrenförmigen Elements (66), um das Einführen des konformen inneren Gehäuses (64) in das starre äußere Gehäuse (62) durch das offene distale Ende des röhrenförmigen Elements (66) zu ermöglichen; und
ein Kragenelement (102), das auf dem länglichen Körper (96) ausgebildet ist und sich radial von dort nach außen erstreckt, wobei der Kragen neben dem geschlossenen distalen Ende des länglichen Körpers (96) positioniert ist;
wobei das Kragenelement (102) mindestens teilweise innerhalb des einen oder mehreren Fenster (86) zurückgehalten wird, um das konforme innere Gehäuse (64) innerhalb des starren äußeren Gehäuses (62) zu sichern.

5. Spritze (10) nach Anspruch 4, wobei der längliche Körper (96) einen darin gebildeten Hohlraum (104) enthält, die sich distal in den länglichen Körper (96) von dessen offenem proximalen Ende erstreckt.

6. Spritze (10) nach Anspruch 5, wobei sich die Nadel (52) mit dem Nadelschutz (60), der in den Spritzenkörper (15) eingreift, distal durch den Hohlraum (104) und in ein Elastomermaterial des konformen inneren Gehäuses (64) erstreckt, das distal zum Hohlraum (104) verläuft.

7. Spritze (10) nach Anspruch 5, wobei der längliche Körper (96) einen am offenen proximalen Ende davon positionierten Abdichtungsring (110) enthält, wobei der Abdichtungsring (110) eine Öffnung des Hohlraums (104) umgibt.

8. Spritze (10) nach Anspruch 7, wobei der Nabenabschnitt (30) einen Durchmesser aufweist, der kleiner ist als ein zylindrischer Hauptabschnitt des Spritzenzylinders (15), der sich distal davon erstreckt, wobei der Nabenabschnitt (30) einschließlich der darauf gebildeten radialen Erhebung (34) einen Kanal (32) in Fluidkommunikation mit der Kammer (20) definiert, wobei die Nadel (52) innerhalb des Kanals (32) gesichert ist, und wobei der Abdichtungsring (110) ein distales Ende des Nabenabschnitts (30) einrastet, um eine Abdichtung mit dem Nabenabschnitt (30) zu bilden und eine weitere proximale Bewegung des Nadelschutzes (60) zu verhindern.

9. Spritze (10) nach Anspruch 8, wobei jeder des Abdichtungsrings (110) und des distalen Endes des Nabenabschnitts (30) eine Paarungsfläche enthält, wobei die Paarungsflächen des Abdichtungsrings (110) und des Nabenabschnitts (30) ineinandergreifen, wobei die Paarungsflächen abgewinkelte Paarungsflächen oder flache Paarungsflächen umfassen.

10. Spritze (10) nach Anspruch 8 oder Anspruch 9, wobei das Eingreifen des Abdichtungsrings (110) in das distale Ende des Nabenabschnitts (30) zusammen mit dem Eingreifen der Nadel (52) in das konforme innere Gehäuse (64) eine distal gerichtete Kraft erzeugt, die das Kragenelement (102) gegen eine distale Kante des einen oder mehreren Fenster (86) nach oben drückt.

11. Spritze (10) nach einem der Ansprüche 7 bis 10, wobei der Abdichtungsring (110) innerhalb des Hauptkörperabschnitts (72) des starren äußeren Gehäuses (62) so positioniert ist, dass der Bruchabschnitt (74) des starren äußeren Gehäuses (62) proximal vom konformen inneren Gehäuse (64) ist.

12. Spritze (10) nach einem der Ansprüche 7 bis 11, wobei der Abdichtungsring (110) des länglichen Körpers (96) axial von der radialen Erhebung (34) getrennt ist.

13. Spritze (10) nach einem der Ansprüche 4 bis 12, wobei der längliche Körper (96) vom geschlossenen distalen Ende zum offenen proximalen Ende verjüngt ist.

14. Spritze (10) nach einem der Ansprüche 1 bis 13, wobei eine Ausziehkraft, die erforderlich ist, um den Nadelschutz (60) vom Spritzenkörper (15) und von der Nadel (52) zu entfernen, gleich einer Kraft ist, die erforderlich ist, um den umlaufenden Buchsteg (76) zu brechen, so dass der umlaufende Bruchsteg (76) beim Entfernen des Nadelschutzes (60) vom Spritzenkörper (15) bricht und der Bruchabschnitt (74) am Spritzenkörper (15) bleibt.

## Revendications

1. Seringue préremplie ou préremplissable (10), la seringue (10) comprenant :
un ensemble de seringue (12) qui comprend :
un cylindre de seringue (15) ayant une extrémité distale de cylindre (24) et une extrémité proximale de cylindre (22) et définissant une chambre (20), le cylindre de seringue (15) comprenant un élément de pointe (18) au niveau de l'extrémité distale de cylindre (24), l'élément de pointe (18) comprenant une partie de raccord (30) ayant une extrémité distale et une extrémité proximale ;
une aiguille (52) ayant une pointe d'aiguille au niveau d'une extrémité distale de l'aiguille (52) et ayant une extrémité proximale insérée de manière fixe dans l'élément de pointe (18) ; et
un protecteur d'aiguille (60) ayant une extrémité distale de protecteur et une extrémité proximale de protecteur, l'extrémité proximale de protecteur étant fixée à l'élément de pointe (18) du cylindre de seringue (15), de manière à être positionnée sur l'aiguille (52) ; et
un ensemble de piston (14) reçu dans la chambre (20) du cylindre de seringue (15) et mobile axialement dans celle-ci ;
dans laquelle le protecteur d'aiguille (60) comprend une enveloppe extérieure rigide (62) et une enveloppe intérieure souple (64) positionnée à l'intérieur de l'enveloppe extérieure rigide (62),
dans laquelle l'enveloppe extérieure rigide (62) comprend une partie frangible inviolable (74) au niveau de l'extrémité proximale de protecteur, la partie frangible (74) étant fixée à une partie de corps principale (72) de l'enveloppe extérieure rigide (62) par une bande frangible circonférentielle (76), et
**caractérisée en ce que** la partie de raccord (30) comprend un bossage radial (34) formé sur celle-ci à un emplacement axialement entre l'extrémité distale et l'extrémité proximale et adjacent à l'extrémité proximale de la partie de raccord (30), le bossage radial (34) présentant une forme en bosse et s'étendant radialement vers l'extérieur à partir du reste de la partie de raccord (30), dans laquelle la partie frangible (74) comprend un système de crochets (88) qui s'engage avec une surface orientée vers le côté proximal (34a) du bossage radial (34), afin d'engager le protecteur d'aiguille (60) avec le cylindre de seringue (15), le système de crochets (88) comprenant une pluralité de crochets (90) formés au niveau d'un bord proximal de la partie frangible (74) et agencés circonférentiellement autour d'une extrémité proximale ouverte (68) de l'enveloppe extérieure (62), la pluralité de crochets (90) étant inclinés de manière distale et s'étendant radialement vers l'intérieur dans une ouverture (94) définie par le bord proximal de la partie frangible (74), les crochets (90) se courbant et glissant au-delà du bossage radial (34) lorsque le protecteur d'aiguille (60) est poussé de manière proximale sur l'aiguille (52) et sur l'élément de pointe (18), et les crochets (90) s'engageant avec la surface orientée vers le côté proximal (34a) du bossage radial (34) pour maintenir le protecteur d'aiguille (60) en place.

2. Seringue (10) selon la revendication 1, dans laquelle l'enveloppe extérieure rigide (62) comprend un élément tubulaire (66) comprenant une extrémité proximale ouverte et une extrémité distale ouverte, la partie de corps principale (72) et la partie frangible (74) étant alignées axialement pour former ensemble l'élément tubulaire (66), et la partie frangible (74) étant positionnée de manière proximale par rapport à la partie de corps principale (72) et attachée à celle-ci par la bande frangible circonférentielle (76).

3. Seringue (10) selon la revendication 2, dans laquelle la partie de corps principale (72) comprend une ou plusieurs fenêtres (86) formées dans celle-ci de manière adjacente à l'extrémité distale ouverte.

4. Seringue (10) selon la revendication 3, dans laquelle l'enveloppe intérieure souple (64) comprend :
un corps allongé (96) ayant une extrémité distale fermée et une extrémité proximale ouverte, le corps allongé (96) ayant un diamètre extérieur inférieur au diamètre intérieur de l'élément tubulaire (66) afin de permettre l'insertion de l'enveloppe intérieure souple (64) dans l'enveloppe extérieure rigide (62) par l'extrémité distale ouverte de l'élément tubulaire (66) ; et
un élément de collier (102) formé sur le corps allongé (96) et s'étendant radialement vers l'extérieur à partir de celui-ci, le collier étant positionné de manière adjacente à l'extrémité distale fermée du corps allongé (96) ;
dans laquelle l'élément de collier (102) est au moins partiellement retenu à l'intérieur de la ou des plusieurs fenêtres (86) afin de fixer l'enveloppe intérieure souple (64) dans l'enveloppe extérieure rigide (62).

5. Seringue (10) selon la revendication 4, dans laquelle le corps allongé (96) comprend une cavité (104) formée dans celui-ci, s'étendant de manière distale dans le corps allongé (96) à partir de son extrémité proximale ouverte.

6. Seringue (10) selon la revendication 5, dans laquelle, lorsque le protecteur d'aiguille (60) est engagé avec le cylindre de seringue (15), l'aiguille (52) s'étend de manière distale à travers la cavité (104) et dans un matériau élastomère de l'enveloppe intérieure souple (64) qui est distal par rapport à la cavité (104).

7. Seringue (10) selon la revendication 5, dans laquelle le corps allongé (96) comprend une bague d'étanchéité (110) positionnée au niveau de son extrémité proximale ouverte, la bague d'étanchéité (110) entourant une ouverture de la cavité (104).

8. Seringue (10) selon la revendication 7, dans laquelle la partie de raccord (30) a un diamètre inférieur à celui d'une partie principale cylindrique du cylindre de seringue (15) s'étendant de manière distale à partir de celle-ci, la partie de raccord (30) comprenant le bossage radial (34) formé sur celle-ci et définissant un canal (32) en communication fluidique avec la chambre (20), l'aiguille (52) étant fixée à l'intérieur du canal (32), et dans laquelle la bague d'étanchéité (110) s'engage avec une extrémité distale de la partie de raccord (30) pour former un joint étanche avec la partie de raccord (30) et empêcher tout mouvement proximal supplémentaire du protecteur d'aiguille (60).

9. Seringue (10) selon la revendication 8, dans laquelle chacune de la bague d'étanchéité (110) et de l'extrémité distale de la partie de raccord (30) comprend une surface d'accouplement, les surfaces d'accouplement de la bague d'étanchéité (110) et de la partie de raccord (30) s'engageant l'une avec l'autre, les surfaces d'accouplement comprenant des surfaces d'accouplement inclinées ou des surfaces d'accouplement plates.

10. Seringue (10) selon la revendication 8 ou la revendication 9, dans laquelle l'engagement de la bague d'étanchéité (110) avec l'extrémité distale de la partie de raccord (30), conjointement avec l'engagement de l'aiguille (52) avec l'enveloppe intérieure souple (64), génère une force dirigée distalement qui pousse l'élément de collier (102) contre un bord distal de la ou des plusieurs fenêtres (86).

11. Seringue (10) selon l'une des revendications 7 à 10, dans laquelle la bague d'étanchéité (110) est positionnée dans la partie de corps principale (72) de l'enveloppe extérieure rigide (62) de sorte que la partie frangible (74) de l'enveloppe extérieure rigide (62) soit proximale par rapport à l'enveloppe intérieure souple (64).

12. Seringue (10) selon l'une des revendications 7 à 11, dans laquelle la bague d'étanchéité (110) du corps allongé (96) est séparée axialement du bossage radial (34).

13. Seringue (10) selon l'une des revendications 4 à 12, dans laquelle le corps allongé (96) est effilé de son extrémité distale fermée à son extrémité proximale ouverte.

14. Seringue (10) selon l'une des revendications 1 à 13, dans laquelle la force d'extraction nécessaire pour retirer le protecteur d'aiguille (60) du cylindre de seringue (15) et de l'aiguille (52) est égale à la force nécessaire pour rompre la bande frangible circonférentielle (76), de sorte que la bande frangible circonférentielle (76) se rompe lors du retrait du protecteur d'aiguille (60) du cylindre de seringue (15), laissant la partie frangible (74) sur le cylindre de seringue (15).
